# EUROPEAN PATENT SPECIFICATION

(11) **EP 4 463 438 B1**
(45) Date of publication and mention of the grant of the patent: **07.01.2026**
(21) Application number: 23701615.9
(22) Date of filing: 12.01.2023
(51) Int. Cl.: C07C 253/34, C07C 255/38

(54) **IMPROVED PROCESS OPTIMIZATION IN PRODUCTION OF AN INSECTICIDAL SUBSTANCE IN INDUSTRIAL SCALE**
VERBESSERTE PROZESSOPTIMIERUNG BEI DER HERSTELLUNG EINER INSEKTIZIDEN SUBSTANZ IM INDUSTRIELLEN MASSSTAB
OPTIMISATION DU PROCÉDÉ AMÉLIORÉ DE PRODUCTION D'UNE SUBSTANCE INSECTICIDE À L'ÉCHELLE INDUSTRIELLE

(30) Priority: 13.01.2022 ZA 202200615
(43) Date of publication of application: 20.11.2024
(73) Proprietor: ADAMA MAKHTESHIM LTD., 8410001 Beer Sheva (IL)
(72) Inventor: PEN, Liza, 8423658 Beer Sheva (IL); ZARCHIN, Ruby, 7850117 Ashkelon (IL); GELMAN, Elijah, 8451598 Beer Sheva (IL)
(74) Representative: Modiano, Gabriella Diana
(86) International application number: PCT/IL2023/050038
(87) International publication number: WO 2023/135599

(56) References cited:
- "Inorganic Anticorrosive Materials", 1 January 2022, ELSEVIER, ISBN: 978-0-32-390410-0, article BANERJEE PRIYABRATA ET AL: "MgO as corrosion inhibitor", pages: 183 - 210, XP093034348, DOI: 10.1016/B978-0-323-90410-0.00011-8
- R. J. ANDERSONK. G. ADAMSC. A. HENRICK: "Synthesis and insecticidal activity of the stereoisomers of a-cyano-3-phenoxybenzyl 2-[2-chloro-4- (trifluoromethyl)anilino]-3-methylbutanoate (fluvalinate) and related analogues", JOURNAL OF AGRICULTURAL AND FOOD CHEMISTRY, vol. 33, no. 3, 1985, pages 508 - 514, XP093034286

## Description

This application claims benefits of a South African Provisional Application Number, 2022/00615 filed on January 13, 2022.

### FIELD OF INVENTION

The present invention relates to an improved process for the preparation of an insecticidal active substance involving controlled optimization of the reaction conditions. More particularly, the invention relates to an industrial-scale production process of Tau-fluvalinate in a purified form comprising optimization of the reaction conditions by timely acid neutralization, resulting in preventing corrosions of the expensive plant equipment.

### BACKGROUND OF INVENTION

Production of active chemical active substances in industrial scale is fraught with number of operational challenges. The challenges which look less significant in a laboratory scale or even in a pilot scale may turn out to be the most significant challenge in industrial-scale production of an active substance, either in terms of operational feasibility or cost-effectiveness or both.

Tau-fluvalinate, [cyano-(3-phenoxyphenyl) methyl] (2R)-2-[2-chloro-4-(trifluoromethyl) anilino]-3-methylbutanoate, a synthetic pyrethroid insecticide is the (2R) diastereomers of fluvalinate.

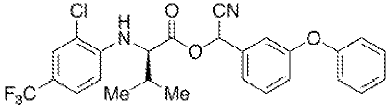

There are many processes of Tau-fluvalinate known in the literature since last few decades. One such literature is R. J. Anderson, K. G. Adams, and C. A. Henrick, "Synthesis and insecticidal activity of the stereoisomers of α-cyano-3-phenoxybenzyl 2-[2-chloro-4-(trifluoromethyl)anilino]-3-methylbutanoate (fluvalinate) and related analogues," Journal of Agricultural and Food Chemistry, vol. 33, no. 3, pp. 508-514, 1985.

Tau-fluvalinate is primarily produced commercially *inter alia* starting from the intermediates, (2-chloro-4-(trifluoromethyl)-phenyl)-D-valinoyl-chloride and 2-hydroxy-2-(3-phenoxyphenyl)acetonitrile esterifying under basic condition in an aprotic solvent system. The advanced intermediate, (2-chloro-4-(trifluoromethyl)-phenyl)-D-valinoyl-chloride is known to have been produced from (2-chloro-4-(trifluoromethyl)phenyl)-D-valine by reacting with phosgene.

In the Industrial scale production of Tau-fluvalinate, although the product undergoes several washes along different stages of the process, the final product remains with variety of impurities. It is impossible to prevent the formation of these impurities as well as to manage them, as its level is very low (^{~}500 ppm), but this level of the impurities in an industrial scale production of Tau-fluvalinate is enough to cause corrosion of the expensive equipment by the HCl released during decomposition of those impurities.

In general, two of the common purification procedures are crystallization and distillation. Since Tau-fluvalinate is a liquid material, it is not possible to purify it through crystallization, and therefore, distillation remains only option of purification. However, Tau-fluvalinate is a relatively high boiling point material and temperature sensitive. Thus, this material could be distilled only at very high vacuum (below 0.1 mbar), and at low residence time. Such high vacuum cannot be reached with ordinary reactors and can only be reached by using very expensive and sophisticated reactor having continuous sequential distillation units. It has been observed that those expensive reactors for distillation in the production plant of Tau-fluvalinate are facing serious damage due to corrosion resulting from release of hydrochloric acid by decomposition of the unavoidable impurities, as mentioned above.

There was therefore an urgent need of preventing corrosion to the construction material of the plant reactor by effective controlling or arresting the release of hydrochloric acid from the process related unavoidable impurities.

There have been some endeavors in the relevant art to control or neutralize the mineral acid, but to ensure that no more acid would be formed even after neutralization, in the later stage of the process from some impurity has never been addressed precisely in the production of Tau-fluvalinate.

In light of the above, there is an attempt in the present invention for an improved process optimization in an industrial-scale production process of Tau-fluvalinate in a purified form comprising optimization of the reaction conditions by timely acid neutralization, resulting in preventing corrosions of the expensive plant equipment.

### SUMMARY OF THE PRESENT SUBJECT MATTER

We have reasonably addressed the challenges as described above as a whole or in part by arriving at a production process of Tau-fluvalinate comprising an improved process optimization in reaction conditions by optimum heating of solution of Tau-fluvalinate as well as timely acid neutralization, resulting in preventing acid-induced corrosions of the expensive plant equipment.

The present invention relates to an optimized production process of Tau-fluvalinate comprising (a) adding a neutralizing agent to a crude solution of Tau-fluvalinate; (b) heating the solution of Tau-fluvalinate at an elevated temperature of 130°C or more; (c) cooling the solution of Tau-fluvalinate; and (d) filtering the resulting salt of the neutralizing agent.

The present invention relates to an optimized production process of Tau-fluvalinate comprising (a) adding to a crude solution of Tau-fluvalinate a neutralizing agent selected from a group comprising MgO and CaO; (b) heating the solution of Tau-fluvalinate at a temperature of 130°C or more for more than 2 hours; (c) cooling the solution of Tau-fluvalinate to about 60⁰C; and (d) filtering the resulting salt of the neutralizing agent.

The present invention also relates to an optimized production process of Tau-fluvalinate comprising (a) adding MgO to a crude Toluene solution of Tau-fluvalinate; (b) heating the Toluene solution of Tau-fluvalinate at 130⁰C for 2 hours; (c) cooling the solution of Tau-fluvalinate to 60⁰C; and (d) filtering the resulting salt of MgO and ensuing acid.

The present invention also relates to an optimized production process of Tau-fluvalinate comprising (a) adding 10 mol % of MgO to a crude Toluene solution of Tau-fluvalinate; (b) heating the Toluene solution of Tau-fluvalinate at 130°C for 2 hours; (c) cooling the solution of Tau-fluvalinate to 60⁰C; and (d) filtering the resulting salt of MgO and ensuing acid.

The present invention further relates to an optimized production process of Tau-fluvalinate comprising (a) adding a neutralizing agent to a crude solution of Tau-fluvalinate; (b) heating the solution of Tau-fluvalinate at an elevated temperature of 130°C or more; (c) cooling the solution of Tau-fluvalinate; (d) filtering the resulting salt of the neutralizing agent; and (e) distilling the filtrate solution under a vacuum.

The present invention relates to an optimized production process of Tau-fluvalinate comprising (a) adding to a crude solution of Tau-fluvalinate a neutralizing agent selected from a group comprising MgO and CaO; (b) heating the solution of Tau-fluvalinate at a temperature of 130°C or more for more than 2 hours; (c) cooling the solution of Tau-fluvalinate to about 60⁰C; (d) filtering the resulting salt of the neutralizing agent; and (e) distilling the filtrate solution at a pressure below 0.1 mbar.

The present invention also relates to an optimized production process of Tau-fluvalinate comprising (a) adding MgO to a crude Toluene solution of Tau-fluvalinate; (b) heating the Toluene solution of Tau-fluvalinate at 130⁰C for 2 hours; (c) cooling the solution of Tau-fluvalinate to 60⁰C; (d) filtering the resulting salt of MgO and ensuing acid; and (e) distilling the filtrate solution at a pressure below 0.1 mbar.

The present invention also relates to an optimized production process of Tau-fluvalinate comprising (a) adding 10 mol % of MgO to a crude Toluene solution of Tau-fluvalinate; (b) heating the Toluene solution of Tau-fluvalinate at 130°C for 2 hours; (c) cooling the solution of Tau-fluvalinate to 60⁰C; (d) filtering the resulting salt of MgO and ensuing acid; and (e) distilling the filtrate solution at a pressure below 0.1 mbar.

### DETAILED DESCRIPTION OF THE PRESENT SUBJECT MATTER

### Definitions

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

Certain exemplary embodiments are described to provide an overall understanding of the principles of the invention disclosed herein. It is assumed that those skilled in the art will understand that the inventive features and methods specifically described herein and illustrated in the experimental section are non-limiting exemplary embodiments and that the scope of the present invention is defined solely by the claims. The features illustrated or described in one exemplary embodiment may be combined with the features of other embodiments. Such modifications and variations are intended to be included within the scope of the present invention.

Reference will now be made in detail to embodiments, which are illustrated in the subsequent paragraphs, wherein reference numerals refer to like elements throughout. In this regard, the present embodiments may have different forms and should not be construed as being limited to the descriptions set forth herein. Accordingly, the embodiments are merely described below to explain aspects of the present disclosure.

Throughout the application, descriptions of various embodiments the term "comprising" is used; however, it will be understood by one of skill in the art, that in some specific instances, an embodiment can alternatively be described using the language "consisting essentially of" or "consisting of." The term "a" or "an" as used herein includes the singular and the plural, unless specifically stated otherwise. Therefore, the terms "a," "an", "one or more" or "at least one" can be used interchangeably in this application.

Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

As used in this application, "optimized production process", "process optimization" or "optimized process" have the same meaning, which is one with best economic significance, less wastage or more environmentally viable. In other words, this is an art of adjusting a process so as to optimize some specified set of parameters in a process without violating some constraint. The most common goals are minimizing cost and maximizing efficiency.

Throughout the application, the term, "neutralizing agent" means an agent, or a group of agents meant to be added to a solution to make the solution system neutral from the acidic condition, thereby keeping pH of the solution at 7.

As used in this application, "mbar" means "millibar", which is a unit for measuring the air pressure in a system. Higher the vacuum, lower would be the value of mbar.

For purposes of better understanding the present teachings and in no way limiting the scope of the teachings, each numerical parameter should be construed in light of the number of reported significant digits and by applying ordinary rounding techniques. In this regard, used of the term "about" herein specifically includes a range from the indicated value, deemed reasonable to a person skilled in the art. In addition, the endpoints of all ranges directed to the same component or property herein are inclusive of the endpoints, are independently combinable, and include all intermediate points and ranges.

### Optimized Process

There is always a challenge to purify active substance in large-scale production in pharmaceutical or agrochemical sciences. The challenge becomes more harder when the last step of purification involves a special distillation procedure where expensive, sophisticated and sensitive (especially acid sensitive) equipment or reactors are involved. During the large-scale production process of Tau-fluvalinate, there was also observed one such major problem, wherein the last stage of distillation cannot be carried out with ordinary reactors and can only be done by using very expensive and sophisticated reactor having continuous sequential distillation units. It has been observed that those expensive reactors for distillation in the production plant of Tau-fluvalinate are facing serious damage due to corrosion resulting from release of hydrochloric acid by decomposition of the process related impurities, formed during known condensation reaction of the intermediates, (2-chloro-4-(trifluoromethyl)-phenyl)-D-valinoyl-chloride and 2-hydroxy-2-(3-phenoxyphenyl)acetonitrile.

In the endeavor to solve this challenge, the inventors have attempted several options to optimize the process of treating the acid emanating from the heat-induced decomposition of the impurity, by using various neutralizing agents, so that all the acid can be treated before the crude solution of Tau-fluvalinate reaches the distillation reactor, thereby preventing any harm to the construction material of the said reactor. It is realized that the most critical part is the stage at which the process related impurities will be decomposed by optimum heating to release all hydrochloric acid emanating from the said impurities, and also treating or neutralizing the acid at the same stage. If it is not done in the right stage under a critically controlled condition, the main aim may not be satisfied, as those impurity might still enter in the distillation reactor and cause the release of acid by decomposition of impurities at higher temperature, thereby harming the construction material of the said reactor.

After several experimentations, inventors surprisingly found an optimized production process of Tau-fluvalinate in a critically controlled condition wherein process related impurities would be decomposed by optimum heating to release all hydrochloric acid emanating from the said impurities, and also neutralizing all the acid content at the same stage, thereby preventing any harm to the construction material of the distillation reactor potentially caused by impurity-induced mineral acid.

In an embodiment, the present invention provides an optimized production process of Tau-fluvalinate comprising (a) adding a neutralizing agent to a crude solution of Tau-fluvalinate; (b) heating the solution of Tau-fluvalinate at elevated temperature of 130°C or more; (c) cooling the solution of Tau-fluvalinate; and (d) filtering the resulting salt of the neutralizing agent.

In a specific embodiment, the present invention provides an optimized production process of Tau-fluvalinate comprising (a) adding to a crude solution of Tau-fluvalinate a neutralizing agent selected from a group comprising MgO and CaO; (b) heating the solution of Tau-fluvalinate at a temperature at 130°C or more for about 2 hours; (c) cooling the solution of Tau-fluvalinate to about 60⁰C; and (d) filtering the resulting salt of the neutralizing agent.

In an embodiment, the present invention provides an optimized production process of Tau-fluvalinate comprising (a) adding MgO to a crude Toluene solution of Tau-fluvalinate; (b) heating the Toluene solution of Tau-fluvalinate at 130°C for 2 hours; (c) cooling the solution of Tau-fluvalinate to 60⁰C; and (d) filtering the resulting salt of MgO and ensuing acid.

In another embodiment, the present invention provides an optimized production process of Tau-fluvalinate comprising (a) adding 10 mol % of MgO to a crude Toluene solution of Tau-fluvalinate; (b) heating the Toluene solution of Tau-fluvalinate at 130°C for 2 hours; (c) cooling the solution of Tau-fluvalinate to 60⁰C; and (d) filtering the resulting salt of MgO and ensuing acid.

In another embodiment, the present invention provides an optimized production process of Tau-fluvalinate comprising (a) adding a neutralizing agent to a crude solution of Tau-fluvalinate; (b) heating the solution of Tau-fluvalinate at an elevated temperature of 130°C or more; (c) cooling the solution of Tau-fluvalinate; (d) filtering the resulting salt of the neutralizing agent; and (e) distilling the filtrate solution under a vacuum.

In another embodiment, the present invention provides an optimized production process of Tau-fluvalinate comprising (a) adding to a crude solution of Tau-fluvalinate a neutralizing agent selected from a group comprising MgO and CaO; (b) heating the solution of Tau-fluvalinate at a temperature of 130°C or more for more than 2 hours; (c) cooling the solution of Tau-fluvalinate to about 60⁰C; (d) filtering the resulting salt of the neutralizing agent; and (e) distilling the filtrate solution at a pressure below 0.1 mbar.

In an embodiment, the present invention provides an optimized production process of Tau-fluvalinate comprising (a) adding MgO to a crude Toluene solution of Tau-fluvalinate; (b) heating the Toluene solution of Tau-fluvalinate at 130°C for 2 hours; (c) cooling the solution of Tau-fluvalinate to 60°C; (d) filtering the resulting salt of MgO and ensuing acid; and (e) distilling the filtrate solution at a pressure below 0.1 mbar.

In an embodiment, the present invention provides an optimized production process of Tau-fluvalinate comprising (a) adding 10 mol % of MgO to a crude Toluene solution of Tau-fluvalinate; (b) heating the Toluene solution of Tau-fluvalinate at 130°C for 2 hours; (c) cooling the solution of Tau-fluvalinate to 60⁰C; (d) filtering the resulting salt of MgO and ensuing acid; and (e) distilling the filtrate solution at a pressure below 0.1 mbar.

The following examples illustrate the practice of the present invention in some of its embodiments but should not be construed as limiting the scope of the invention.

### PREPARATORY EXAMPLES:

### A) Process for Tau-fluvalinate:

The Schematic steps for the preparation of Tau-fluvalinate are summarized as follows:

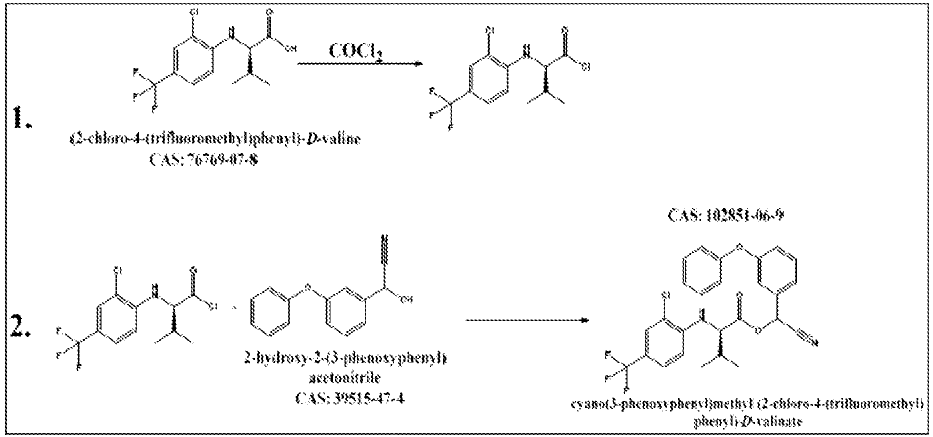

The key intermediate, (2-chloro-4-(trifluoromethyl)phenyl)-D-valinoyl-chloride was prepared from the precursor, (2-chloro-4-(trifluoromethyl)phenyl)-D-valine by reacting with phosgene following the known method described in the literature, such as in US 4,464,307 patent. The subsequent step (step 2) of esterification of (2-chloro-4-(trifluoromethyl)phenyl)-D-valinoyl-chloride with 2-hydroxy-2-(3-phenoxyphenyl)acetonitrile in Toluene/ hexane solvent mixture in presence of base produces viscous oil of Tau-fluvalinate, the detailed process of which is also known in the literature such as in US 4,243,819 patent.

### B) Impurities Decomposition and acid treatment under controlled condition:

The process related impurities present in crude Tau-fluvalinate are decomposed and ensuing mineral acid is neutralized under controlled and critically optimized condition in the following manner:
To a 1000 gr of 50% toluene solution of tau-fluvalinate 4 gr of MgO is added. This solution was heated at 130°C for about 2h and 50% of the toluene was distilled during this heating stage. After the completion of the heating the solution is cooled to 60⁰C. The magnesium salt in the solution is subsequently filtered. The filtrate solution of Tau-fluvalinate is seen to be completely free of acid and no further formation of acid from the decomposition of process related impurities is observed.

It has been further observed that the minimum temperature is 130°C for about 2h, otherwise the impurity decomposition will not be completed and that may result in decomposition during the subsequent stages causing release of acid followed by harming construction material of plant reactors.

### C) Distillation:

After the said acid treatment, about 75% solution of Tau-fluvalinate in toluene was transferred to a sequential distillation path where temperatures are gradually increased by maintaining high vacuum throughout, ultimately at a pressure below 0.1 mbar. Tau-Fluvalinate in assay of 92-94% is obtained without any damage to the expensive distillation reactors in the production plant of Tau-fluvalinate.

## Claims

1. An optimized production process of Tau-fluvalinate comprising steps of
(a) adding an effective amount of a neutralizing agent to a crude solution of Tau-fluvalinate;
(b) heating the solution of Tau-fluvalinate at an elevated temperature of 130°C or more;
(c) cooling the solution of Tau-fluvalinate; and
(d) filtering the resulting salt of the neutralizing agent.

2. The optimized production process of Tau-fluvalinate according to claim 1, wherein in step (a) the neutralizing agent is selected from a group comprising MgO and CaO.

3. The optimized production process of Tau-fluvalinate according to claim 1, wherein the step (b) is heating the solution of Tau-fluvalinate at temperature of 130°C or more for about 2 hours, and in step (c) the cooling temperature is about 60°C.

4. The optimized production process of Tau-fluvalinate according to any claim of claims 1 to 3, comprising steps of
(a) adding an effective amount of MgO to a crude Toluene solution of Tau-fluvalinate;
(b) heating the Toluene solution of Tau-fluvalinate at 130°C for 2 hours;
(c) cooling the solution of Tau-fluvalinate to 60⁰C; and
(d) filtering the resulting salt of MgO and ensuing acid.

5. The optimized production process of Tau-fluvalinate according to claim 4, wherein in step (a) the neutralizing agent is 10 mol % of MgO.

6. The optimized production process of Tau-fluvalinate according to any claim of claims 1 to 5 further comprising a step of (e) distilling the filtrate solution under a vacuum.

7. The optimized production process of Tau-fluvalinate according to claim 6 comprising the step of (e) distilling the filtrate solution at a pressure below 0.1 mbar.

## Patentansprüche

1. Optimierter Herstellungsprozess für Tau-Fluvalinat, umfassend die folgenden Schritte
(a) Zugabe einer wirksamen Menge eines Neutralisationsmittels zu einer Rohlösung von Tau-Fluvalinat;
(b) Erhitzen der Tau-Fluvalinat-Lösung auf eine erhöhte Temperatur von 130 °C oder mehr;
(c) Abkühlen der Tau-Fluvalinat-Lösung; und
(d) Filtern des resultierenden Salzes des Neutralisationsmittels.

2. Optimiertes Herstellungsverfahren für Tau-Fluvalinat gemäß Anspruch 1, wobei in Schritt (a) das Neutralisationsmittel aus einer Gruppe ausgewählt wird, die MgO und CaO umfasst.

3. Optimiertes Herstellungsverfahren für Tau-Fluvalinat gemäß Anspruch 1, wobei in Schritt (b) die Tau-Fluvalinat-Lösung etwa 2 Stunden lang auf einer Temperatur von 130 °C oder mehr erhitzt wird und in Schritt (c) die Abkühltemperatur etwa 60 °C beträgt.

4. Optimiertes Herstellungsverfahren für Tau-Fluvalinat gemäß einem der Ansprüche 1 bis 3, umfassend die Schritte
(a) Zugabe einer wirksamen Menge MgO zu einer rohen Toluollösung von Tau-Fluvalinat;
(b) Erhitzen der Toluollösung von Tau-Fluvalinat bei 130 °C für 2 Stunden;
(c) Abkühlen der Lösung von Tau-Fluvalinat auf 60 °C; und
(d) Filtrieren des resultierenden Salzes aus MgO und der entstehenden Säure.

5. Optimiertes Herstellungsverfahren für Tau-Fluvalinat gemäß Anspruch 4, wobei in Schritt (a) das Neutralisationsmittel 10 Mol-% MgO beträgt.

6. Optimiertes Herstellungsverfahren für Tau-Fluvalinat gemäß einem der Ansprüche 1 bis 5, das ferner einen Schritt (e) des Destillierens der Filtratlösung unter Vakuum umfasst.

7. Optimiertes Herstellungsverfahren für Tau-Fluvalinat gemäß Anspruch 6, das den Schritt (e) des Destillierens der Filtratlösung bei einem Druck unter 0,1 mbar umfasst.

## Revendications

1. Procédé optimisé de production de tau-fluvalinate comprenant les étapes suivantes
(a) ajouter une quantité efficace d'un agent neutralisant à une solution brute de tau-fluvalinate ;
(b) chauffer la solution de tau-fluvalinate à une température élevée de 130 °C ou plus ;
(c) refroidir la solution de tau-fluvalinate ; et
(d) filtrer le sel résultant de l'agent neutralisant.

2. Procédé de production optimisé de tau-fluvalinate selon la revendication 1, où, à l'étape (a), l'agent neutralisant est choisi parmi un groupe comprenant MgO et CaO.

3. Procédé de production optimisé du tau-fluvalinate selon la revendication 1, où l'étape (b) consiste à chauffer la solution de tau-fluvalinate à une température de 130 °C ou plus pendant environ 2 heures, et dans l'étape (c), la température de refroidissement est d'environ 60 °C.

4. Procédé de production optimisé du tau-fluvalinate selon l'une quelconque des revendications 1 à 3, comprenant les étapes suivantes
(a) ajouter une quantité efficace de MgO à une solution brute de toluène de tau-fluvalinate ;
(b) chauffer la solution de toluène de tau-fluvalinate à 130 °C pendant 2 heures ;
(c) refroidir la solution de tau-fluvalinate à 60 °C ; et
(d) filtrer le sel de MgO et l'acide qui en résulte.

5. Procédé de production optimisé du tau-fluvalinate selon la revendication 4, où, à l'étape (a), l'agent neutralisant est constitué de 10 % en moles de MgO.

6. Procédé de production optimisé du tau-fluvalinate selon l'une quelconque des revendications 1 à 5, comprenant en outre une étape (e) de distillation de la solution filtrée sous vide.

7. Procédé de production optimisé du tau-fluvalinate selon la revendication 6, comprenant l'étape (e) de distillation de la solution filtrée à une pression inférieure à 0,1 mbar.
